# EUROPEAN PATENT APPLICATION

(11) **EP 2 404 642 A2**
(43) Date of publication of application: **11.01.2012**
(21) Application number: 11172209.6
(22) Date of filing: 30.06.2011
(51) Int. Cl.: A61Q 19/08, A61K 8/65, A61K 8/67, A61K 8/97

(54) **Process for preparing a cosmetic composition having an immediate lifting effect**

(30) Priority: 09.07.2010 IT MI20101271
(71) Applicant: The Direct Marketing Company S.p.A. (brevemente DMC S.p.A.), 47891 Rovereta (SM)
(72) Inventor: Di Filippo, Fernando, 47891 Rovereta (SM)
(74) Representative: Petruzziello, Aldo

(57) **Abstract**

A description is given of a process for preparing a dermo-cosmetic composition having an instant lifting effect in serum/gel form comprising, as main active ingredients, vitamin A, vitamin E derivatives, in particular tocopheril acetate, collagen, ascorbic acid in combination with a mixture of plant extracts of *Spilanthes Acmella Flower; Cucumus Sativus, Olea Europaea Leaf, Punica Granatum, Gingko Biloba, Vitis Vinifera Leaf,* in at least one physiologically acceptable medium or carrier.

## Description

The present invention relates to a dermo-cosmetic composition with an immediate lifting effect for the treatment of the unsightly effects of skin ageing and to the method of preparation of said composition.

More particularly the present invention relates to a composition, as defined above, with a tightening effect on the skin of the face, neck and hands only 3-5 minutes after application.

The skin constantly undergoes a natural process of renewal, constituted by the continuous elimination of dead cells and by the production of new cells. This natural cycle of regeneration slows down with advancing age, the skin produces fewer proteins, the dermis becomes thinner and the loss of elasticity causes the appearance of small wrinkles and the fading of the radiance of the face.

To combat these various signs the market has made available a large variety of preparations for the care and treatment of the body for topical application: however most of these formulations for anti-ageing creams contain particular components able to perform a specific action on a specific area to respond to its particular problem, for example only the area around the eyes or only the area around the mouth.

Many of the various known formulations also have disadvantages linked to their intrinsic features: stickiness, discomfort after treatment, for example due to skin dryness, presence of fragrance or alcohol, skin reddening and an insignificant tightening effect, etc.

In particular these classic treatment formulations very often do not show the visibly immediate results of a tightening effect since their efficacy in short-term treatment is often not rapid.

Most of the products on sale do not then have clinical studies to support the efficacy, safety and results, nor are they even cited in the more authoritative scientific and/or medical journals.

Last but not least the financial aspect, since all dermo-cosmetic treatments currently present on the market provide for prolonged applications in time by means of repeated cycles of applications before having good efficacy, with huge overall cost: improving personal appearance without spending huge sums, obtaining visible yet natural results, is an actual need.

There is therefore the need for the availability of a cosmetic composition which responds to what is stated above and which the market leaders currently do not offer.

The main object of the present invention is that of having availability of a dermo-cosmetic product with an immediate tightening (lifting) effect, able to overcome the disadvantages of the prior art, which is effective on face, neck and back of the hands after its first application and effective in short-term treatment, with aesthetically valid and visible results already after a few minutes and which at the same time is easy to administer.

Another object is that of providing such a product which is free from stickiness and from alcohol and which does not cause discomfort after treatment for example due to skin dryness or skin reddening.

Yet another object is that of providing a product which is easy to prepare and economical.

These objects, and others, which will be made clearer by the following detailed description, were achieved by the Applicant who has found that a particular selection of active ingredients shows, mainly, an unexpected tightening effect.

Therefore a first object of the present invention is formed by a dermo-cosmetic composition having the features as disclosed in the attached independent claim.

The present composition with an immediate lifting effect comprises, as main active ingredients, vitamin A (in particular retinyl palmitate), vitamin E, pure or in its other forms (in particular tocopheril acetate), collagen, ascorbic acid in combination with a mixture of plant extracts such as *Spilanthes Acmella Flower, Cucumus Sativus, Olea Europaea Leaf, Punica Granatum, Gingko Biloba, Vitis Vinifera Leaf,* said ingredients being present in at least one physiologically acceptable medium or carrier, preferably water.

The combined use of said active ingredients and the present preparation process, which allows dispersion of said active ingredients in the carrier in an efficient manner, allow a dermo-cosmetic composition to be obtained with the capacity to restore to the skin of the face and of the hands an immediately younger and more attractive appearance: in a few minutes, generally 3 to 5 minutes, the skin is smoother and distended, less wrinkled and with a visible improvement in the signs of photo-ageing.

The dermo-cosmetic composition which is the object of the present invention has been tested clinically via a series of technical and instrumental measurements and valuations on a sample of men and women (see the examples).

The instrumental measurements performed highlighted the fact that the application of the product led to a significant decrease in the parameters of skin roughness Ra (mean roughness) and Rz (maximum roughness) at the wrinkles around the eyes, the wrinkles around the lips and on the back of the hands.

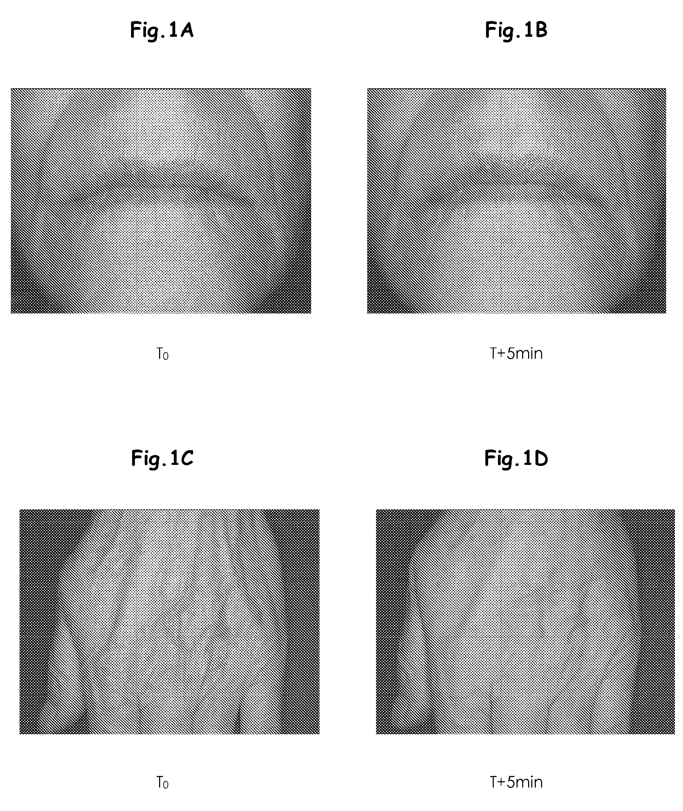

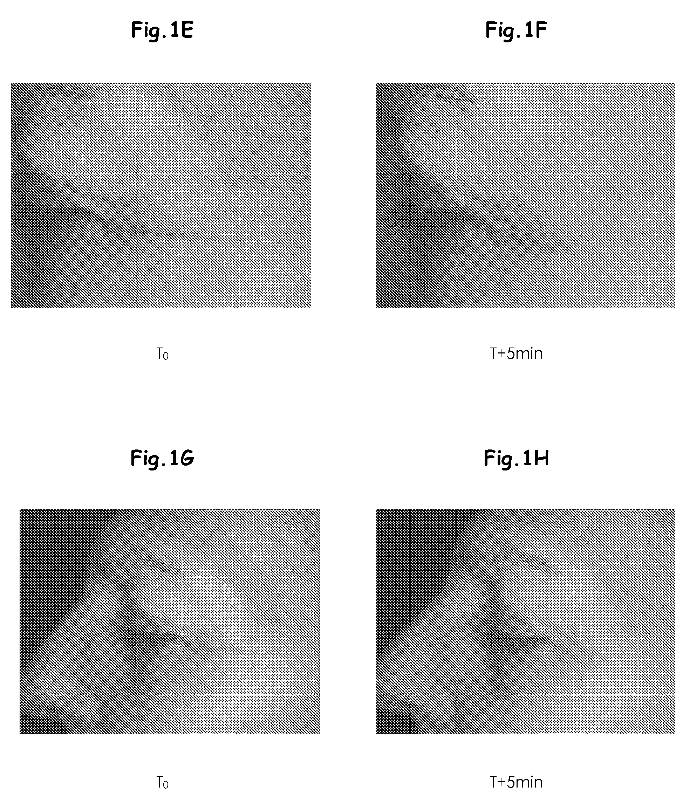

The immediate lifting effect of the present product was also highlighted by a visible improvement (decrease) in the skin roughness which can be clearly appreciated from the digital images (see Figs. 1-2 which show some photographic images of skin zones treated with the composition of the present invention and with a known composition with lifting effect).

A method for the preparation of a dermo-cosmetic composition having the features as stated herein below forms a further object of the present invention.

In the present invention the quantities are expressed in % by weight with respect to the total weight of the composition.

Moreover, if not indicated, the compounds/ingredients of the composition, the object of the present invention, are to be considered as chosen from among the compounds/ingredients commonly used by persons skilled in the cosmetic art.

All the names given in italics comply with INCI-EU nomenclature.

The components denoted "(a.i.)" represent the active components or ingredients of the reference composition.

The process of the present invention, suitable for the preparation of a semifluid aqueous serum/gel, comprises:
- preparation of a colloidal gel by means of dispersion in water of a powdery mixture of *Cellulose Gum* and *Magnesium Aluminum Silicate*
- addition to this colloidal gel (dispersion), while stirring, of
   a) a mixture comprising vitamin A, vitamin E, pure or in its other forms, extract of *Spilanthes Acmella Flower,* dissolved in a solvent formed by a mixture of propylene glycol, ethylparaben and methylparaben,
   b) an aqueous solution of ascorbic acid,
   c) an aqueous dispersion of *Sodium Silicate,*
- addition of the remaining active ingredients.

More particularly the process of the present invention comprises the following phases:

### STEP 1: the powders of

- *Cellulose Gum,* in an adequate degree of polymerisation, and therefore of viscosity, in adequate degree of grain size and degree of substitution (solubility), in amounts between 0.7% and 1.5%;
- *Magnesium Aluminum Silicate,* also known as "natural smectite clay" or bentonite, with appropriate ratio of aluminium and magnesium, in amounts between 0.5-1.50%, preferably between 0.8% and 1.3;
   are mixed one with the other in order to obtain a homogeneous mixture in powdery form. Mixing is advisable to be repeated at least 2 times with rest times varying between 30 and 60 minutes. It is necessary to operate in such a way as to avoid even partial absorption of humidity.

An example of adequate viscosity of cellulose gum may be that comprised within the range of 400-1500 mPa's; an example of adequate degree of grain size may be that of having a maximum residue of 1% on 0.6 mm screen and a maximum residue of 10% on 0.425 mm screen; an example of adequate degree of substitution may be a value comprised within the range of 0.80-0.95.

An example of appropriate ratio of aluminium and magnesium in the *Magnesium Aluminum Silicate* may be a value comprised between 0.3-1.3.

### STEP 2:

- Heat a first portion of water, generally a quantity varying between 70% and 80% in relation to the quantity of total water in the final composition, and bring the working temperature of said portion of water within the range between 70°-80°;
- in the quantity of water indicated above introduce slowly, preferably by sprinkling, the powdery mixture obtained in STEP 1, continuing to stir constantly, preferably with an intensity of stirring of 700-900 rpm and for a mixing time between 35-50 minutes, until obtaining a colloidal gel.

The powdery mixture obtained in STEP 1 has to be correctly dispersed in water and hydrated to obtain the best performances. In this STEP 2 other materials present in the water are to be avoided, including preservatives, chelating agents or other additives in smaller quantities which may interfere with and inhibit the formation of the colloidal structure.

In the preparation of STEP 2 it is advisable to pay attention to the correct degree of hydration which is directly proportional to the quantity of energy used to disperse the phase. It increases in a manner directly proportional to the following factors: time of mixing, intensity of stirring, temperature of the water.

Different solutions/dispersions, prepared in the following steps according to the following procedure, will be added to this colloidal gel obtained in STEP 2.

### STEP 3:

- heat the *Propylene Glycol* in an appropriate steel container to a temperature between 40-60° in a quantity between 6% and 8%, preferably between 7.5% and 8%, and introduce while constantly stirring the *Ethyl parahydroxybenzoate,* also known as ethylparaben, in a quantity between 0.05-0.15%, preferably between 0.08 and 0.13%, and the *Methyl parahydroxybenzoate,* also known as methylparaben, in a quantity between 0.15 and 0.3%, preferably between 0.15 and 0.25%.

Cool the mixture to ambient temperature at complete limpidity of the phase and solubilisation of the two components in the propylene glycol.

### STEP 4:

Slowly introduce into the mixture of STEP 3 each of the following components while constantly stirring:
- *Tocopheryl Acetate* (a.i.) in an amount between 0.05% and 0.15%, preferably between 0.1 and 0.15%,
- *Retinyl Palmitate* (a.i.) in an amount between 0.08 and 0.20%, preferably between 0.08 and 0.14%,
- *Caprylic*/*Capric Triglyceride,* in an amount between 0.001 and 0.020%, preferably between 0.001 % and 0.002%,
- *Spilanthes Acmella Flower Extract* (Asteracee family) (a.i.) in an amount between 0.001 and 0.020%, preferably between 0.001% and 0.002%,
   checking after each addition that the components have solubilised.

### STEP 5:

Bring the colloidal gel of STEP 2 to ambient temperature and introduce, while constantly stirring, STEP 4.

Make sure the right intensity of stirring is applied to avoid breaking the colloidal gel formed in STEP 2.

### STEP 6:

Dissolve the ascorbic acid (a.i.) in an amount between 0.08 and 0.15%, preferably between 0.09 and 0.14%, in the quantity of water remaining with respect to the % of total water of the final composition.

Once solubilisation has taken place, add, while constantly stirring, this STEP 6 to STEP 2.

### STEP 7:

Heat a second portion of water, generally a quantity varying between 10% and 20% in relation to the total water in the formula, and bring the working temperature to around 35-40°.

Add *Sodium Silicate* powder in an amount between 3 and 5.50%, preferably 4 and 5.50%, until complete solubilisation. Cool to ambient temperature.

### STEP 8:

Add, while constantly stirring, the mixture of STEP 7 to STEP 2. Deaerate the product if necessary.

At this point the remaining components of the formulation are subsequently added to the mixture of step 2, to which the aforementioned ingredients have been added, according to the following procedure:

### STEP 9:

Add slowly to the so obtained STEP 2, while stirring and under vacuum, preferably high vacuum, the following components:
- *Soluble Collagen* (a.i.) in an amount between 0.08 and 0.15%, preferably between 0.09 and 0.15%,
- *Cucumis Sativus Extract* (a.i.), in an amount between 0.01 and 0.15%, preferably between 0.09 and 0.15%,
- *Vitis Vinifera Leaf Extract* (a.i.), in an amount between 0.01 and 0.10%, preferably between 0.03 and 0.10%,
- *Ginkgo Biloba Extract* (a.i.), in an amount between 0.01 and 0.15%, preferably between 0.05% and 0.15%,
- *Olea Europaea Leaf Extract* (a.i.), in an amount between 0.01 and 0.2%,
- *Punica Granatum Extract* (a.i.), in an amount between 0.01 and 0.2%, preferably between 0.07 and 0.18%.

It should be noted that one or more of the active ingredients (a.i.) used in the composition of the present invention may be added in the form of a mixture with glycerin.

At the end of this preparation procedure a dermo-cosmetic composition is obtained having the form of a semifluid aqueous serum/gel.

The Applicant has found a new and original combination of active ingredients *(Spilanthes Acmella Flower Extract, Cucumus Sativus Extract, Olea Europaea Leaf Extract Punica Granatum Extract, Gingko Biloba Extract, Vitis Vinifera Leaf Extract, Tocopheryl Acetate, Retinyl Palmitate, Soluble Collagen, Ascorbic Acid*) which are suitable for being used in the dermo-cosmetic preparation which is the object of the present invention.

In practice the Applicant has surprisingly found that the use of the aforementioned active ingredients and the process of dispersion of the same in the carrier allow to obtain a formulation which shows a tightening effect, understood as an action of decrease in roughness at the wrinkles around the eyes, the wrinkles around the lips and the back of the hands, in a very short time, already only 3-5 minutes after the application.

This tightening effect is improved and superior to one of the market leader products (Rilastil Instant Serum).

In addition to the tightening effect, the present composition also shows a hydrating and anti-ageing power with efficacy both in the short term and in the medium-long term, and thanks to the richness of powerful active ingredients also shows a smoothing, antioxidant/anti-free-radicals, purifying and soothing effect both in the short and in the medium-long term, with a visible improvement in the signs of photo-ageing.

In an embodiment of the present invention the composition has the following formula:

| **INCI** | **Quantity %** |
|---|---|
| Aqua | q.b. |
| Propylene Glycol | 7.50 - 8.00 |
| Sodium Silicate | 4.00 - 5.50 |
| Cellulose Gum | 0.80 - 1.50 |
| Magnesium Aluminum Silicate | 0.50 - 1.50 |
| Methylparaben | 0.15 - 0.25 |
| Ascorbic Acid | 0.08 - 0.15 |
| Cucumis Sativus Extract | 0.08 - 0.15 |
| Ethylparaben | 0.05 - 0.15 |
| Retinyl Palmitate | 0.08 - 0.20 |
| Tocopheryl Acetate | 0.05 - 0.15 |
| Glycerin | 0.05 - 0.20 |
| Caprylic/Capric Triglyceride | 0.001 - 0.020 |
| Ginkgo Biloba Extract | 0.05 - 0.15 |
| Olea Europaea Leaf Extract | 0.07 - 0.18 |
| Punica Granatum Extract | 0.08 - 0.18 |
| Vitis Vinifera Leaf Extract | 0.03 - 0.10 |
| Soluble Collagen | 0.08 - 0.15 |
| Spilanthes Acmella Flower Extract | 0.001 - 0.020 |

An example of specific formulation of the cosmetic composition of the present invention may be the following:

| **INCI** | **Quantity %** |
|---|---|
| | |
| Aqua | 84.20280 |
| Propylene Glycol | 7.80000 |
| Sodium Silicate | 5.28000 |
| Cellulose Gum | 0.85000 |
| Magnesium Aluminum Silicate | 0.80000 |
| Methylparaben | 0.20000 |
| Ascorbic Acid | 0.09500 |
| Cucumis Sativus Extract | 0.01400 |
| Ethylparaben | 0.09000 |
| Retinyl Palmitate | 0.08000 |
| Tocopheryl Acetate | 0.15000 |
| Glycerin | 0.08000 |
| Caprylic/Capric Triglyceride | 0.00120 |
| Ginkgo Biloba Extract | 0.05500 |
| Olea Europaea Leaf Extract | 0.01100 |
| Punica Granatum Extract | 0.11000 |
| Vitis Vinifera Leaf Extract | 0.09000 |
| Soluble Collagen | 0.09000 |
| Spilanthes Acmella Flower Extract | 0.00100 |
| | 100.00000 |

Numerous detail changes and modifications may be made to the present embodiment of the invention within the reach of a person skilled in the art, in any case coming within the scope of the invention expressed by the accompanying claims.

Some non-limiting examples of the present invention follow.

### EXAMPLES

### CHARACTERISATION

The tightening effect of the composition in accordance with the invention was valuated by an *in vivo* test, while the quality of said composition was valuated by means of trials and tests performed in accordance with the methods of the certified quality management system (free alkalinity, challenge test, HET-CAM test, dermatological patch test, in vitro irritation test, hypo-allergenic patch test, determination of heavy metals (nickel, chrome and cobalt)), following the general principles of good laboratory practice (GLP) and good clinical practice (GCP), and according to the principles defined in the World Medical Association Declaration of Helsinki, referring to the following references for the *in vivo* tests:
- S. Seidenari: "DIAGNOSTICA NON INVASIVA IN DERMATOLOGIA"; published by EDRA (1998);
- V. Zuang: "THE USE OF NON-INVASIVE TECHNIQUES ON HUMAN VOLUNTEERS TO DETERMINE THE SAFETY AND EFFICACY OF COSMETIC PRODUCTS"; a thesis submitted to the University of Nottingham for the degree of doctor of philosophy, (1999);
- S. A. Glantz: "STA TISTICA PER DISCIPLINE BIO-MEDICHE", McGraw-Hill Libri Italia, second ed.;
- J. Serup, G.B.E. Jemec: "HANDBOOK OF NON-INVASIVE METHODS AND THE SKIN"; CRC Press, Inc., (1995);
- GUIDELINES FOR THE EVALUATION OF THE EFFICACY OF COSMETIC PRODUCTS - COLIPA Guidelines, May 2008;
- JL. Leveque: "EEMCO GUIDANCE FOR THE ASSESSMENT OF SKIN TOPOGRAPHY", J. Eur. Acad. Dermatol. Venereol.; 12(2): 103-114 (Mar. 1999);).

The data given here refer to the sample tested.

The immediate lifting effect was valuated both by instrumental and photographic valuation of the skin relief (skin replica) performed by the special Quantilines image analysis program, Monaderm, based on the principle of measurement described by Corcuff, (P. Corcuff, J.L. Leveque "SKIN SURFACE REPLICA IMAGE ANALYSIS OF FURROWS AND WRINKLES" in J. Serup, G.B.E. Jemec: "HANDBOOK OF NON-INVASIVE METHODS AND THE SKIN"; CRC Press, Inc., (1995*))* for the determination of mean and maximum roughness.

The anti-wrinkle efficacy is demonstrated by a decrease in one or both of the abovementioned parameters at the end of the treatment.

### - SUBJECTS

27 volunteers were used, of whom 24 women and 3 men. The selection was carried out according to the criteria of inclusion (Caucasian race, 8 women aged between 50 and 55 years; 8 women aged between 56 and 60 years; 8 women aged between 61 and 65 years; 3 men aged 55, 60 and 65 years old; subjects having evident wrinkles on the face, in particular in the area around the eyes and around the lips and photo-ageing signs on the hands, with problems of bags and circles, having wrinkles around the eyes yet without pathologies in the period immediately preceding the trials) and of exclusion (subjects undergoing topical or systemic treatment with any drug which may influence the outcome of the test, pregnant or breast-feeding women, subjects affected by skin disorders, subjects with past history of intolerance towards medicines and/or cosmetic products).
- The basal and final instrumental measurements were performed inside a climate-controlled chamber (24 ± 2 °C ; 50 ± 10 % rh).
- ANALYSIS OF THE IMAGE, Monaderm

To obtain a skin replica, i.e. a negative imprint of the skin surface, the following materials are used:
a fast-hardening synthetic polymer (SILFLO - Flexico Ltd, United Kingdom) double-adhesive discs (3M, 24x40).

The adhesive disc is placed on the skin so as to delimit the skin area to be investigated and to avoid possible stretching of the skin surface during the application of the polymer. A small quantity of polymer is activated by addition of the catalyst and distributed inside the circular area. A few minutes after the application the resin is completely dry and is removed, obtaining the replica of the skin surface considered.

Each negative replica of the skin surface is illuminated by an incident light with an angle of 35° in order to generate shadows: the wider the furrows, the higher the ridges.

The main wrinkle is positioned perpendicularly to the plane of incidence of the light. The image of the surface inspected, equal to 12 x 9 mm, is acquired by means of a video camera (high performance CCD camera, COHU).

The software allows the following parameters to be calculated:
Ra = mean roughness (arithmetic)
Rz = maximum roughness (deep wrinkles)

The anti-wrinkle efficacy is demonstrated by a decrease in one or both of the abovementioned parameters Ra, Rz at the end of the treatment.

With the system FotoFinder Dermoscope Ver. 2.0 it was possible to acquire, memorise and process images of the skin surface before and after the treatment with the composition of the invention.

The system consisted of a high-definition colour video camera which allows every surface to be enlarged by means of a series of lenses.

The digital images are displayed on the screen in their real colours. This allows the observer to appreciate even the smallest details of the image. The software also has a function which allows the images acquired to be compared with the reference images filed by the user.

### - MATHEMATICAL PROCESSING OF THE RESULTS

On the basis of the results of the normality tests (Kolmogorov-Smirnov test, Lilliefors test and Shapiro-Wilk test), the data are considered parametric.

For each of the series of values (initial and final) relating to the skin replicas the mean value and the standard deviation were calculated.

The variation of the values for every parameter and the percentage of variation were also calculated.

The instrumental values recorded at the two control times (start and end) were compared statistically by t-test for dependent samples.

The data were considered statistically different for a value of probability p ≤ 0.05.

The efficacy of the product is demonstrated by the significant decrease in the values of skin roughness and by a visible improvement in the signs of photo-ageing.

### Example 1

The product in accordance with the present invention in the form of a beige fluid was applied on the skin of the face and on the back of the hands of the sample of subjects, not previously treated with any cosmetic, of volunteers, valuating the lifting effect thereof immediately after its application.

Before the application of the product the skin replicas of the area around the eyes, around the lips and the back of the hand were carried out on the female volunteers.

The replicas were performed on the 3 male subjects only at the area around the eyes and the back of the hand.

The skin replicas and the digital photos were performed at the start and 5 minutes after the application of the product.

Digital images of the skin areas selected were also taken with the same methods.

After the basal measurements the operator applied an exact quantity of product according to the following methods:
- apply 2 or at most 3 drops of product on all the face (from the hairline to the neck) and 1 drop on the back of the hand;
- spread the serum over the eyelids, under the eyes, around the lips and in the areas where wrinkles are present;
- apply delicately without rubbing;
- leave to dry for 2 or 3 minutes.
5 minutes after the first application of the product the measurements of roughness by means of an imprint and the digital photos of the areas subjected to the test were repeated.

The results given are divided into analysis of the image.

### Area around the eyes (women)

### Ra (Mean roughness)

A significant decrease in the values of mean roughness was recorded at the area around the eyes, 5 minutes after application of the product.

| T₀ | T₅ₘᵢₙ | Variation | % Variation | t-test T₀ vs T₅ₘᵢₙ |
|---|---|---|---|---|
| mean 17.39 *std. dev. 2.29* | mean 13.84 *std. dev. 2.42* | -3.55 | - 20.4% | p < 0.0001 |

### Rz (maximum roughness)

A significant decrease in the values of maximum roughness was recorded at the area around the eyes 5 minutes after application of the product.

| T₀ | T₅ₘᵢₙ | Variation | % Variation | t-test T₀ vs T₅ₘᵢₙ |
|---|---|---|---|---|
| mean 80.68 *std. dev. 7.39* | mean 61.85 *std. dev. 15.10* | -18.83 | -23.3% | p < 0.0001 |

### Area around the lips (women)

### Ra (Mean roughness)

A significant decrease in the values of mean roughness was recorded in the area around the lips 5 minutes after application of the product.

| T₀ | T₅ₘᵢₙ | Variation | % Variation | t-test T₀ vs T₅ₘᵢₙ |
|---|---|---|---|---|
| mean 26.07 *std. dev. 3.3.2* | mean 21.58 *std. dev. 3.36* | -4.49 | -17.2% | p < 0.0001 |

### Rz (maximum roughness)

A significant decrease in the values of maximum roughness was recorded in the area around the lips 5 minutes after application of the product.

| T₀ | T₅ₘᵢₙ | Variation | % Variation | t-test T₀ vs T₅ₘᵢₙ |
|---|---|---|---|---|
| mean 101.70 *std. dev. 9.16* | mean 88.83 *std. dev. 7.81* | - 12.87 | -12.6% | p < 0.0001 |

### Back of the hands (women)

### Ra (mean roughness)

A significant decrease in the values of mean roughness was recorded in the area of the back of the hands 5 minutes after application of the product.

| T₀ | T₅ₘᵢₙ | Variation | % Variation | t-test T₀ vs T₅ₘᵢₙ |
|---|---|---|---|---|
| mean 23.63 *std. dev. 3.97* | mean 17.11 *std. dev. 5.34* | -6.52 | - 27.6% | p < 0.0001 |

### Rz (maximum roughness)

A significant decrease in the values of maximum roughness was recorded in the area of the back of the hands 5 minutes after application of the product.

| T₀ | T₅ₘᵢₙ | Variation | % Variation | t-test T₀ vs T₅ₘᵢₙ |
|---|---|---|---|---|
| mean 107.20 *std. dev. 15.54* | mean 77.89 *std. dev. 25.30* | -29.31 | - 27.3% | p < 0.0001 |

### Area around the eyes (men)

### Ra (mean roughness)

| T₀ | T₅ₘᵢₙ | Variation | % Variation |
|---|---|---|---|
| mean 19.44 *std. dev. 1.60* | mean 18.00 *std. dev. 3.69* | -1.44 | -7.4% |

### Rz (maximum roughness)

| T₀ | T₅ₘᵢₙ | Variation | % Variation |
|---|---|---|---|
| mean 86.64 *std. dev. 10.55* | mean 79.54 *std. dev. 14.08* | - 7.30 | -8.4% |

### Back of the hands (men)

### Ra (mean roughness)

| T₀ | T₅ₘᵢₙ | Variation | % Variation |
|---|---|---|---|
| mean 27.80 *std, dev, 2.00* | mean 21.21 *std. dev. 5.44* | - 6.59 | - 23.7% |

### Rz (maximum roughness)

| T₀ | T₅ₘᵢₙ | Variation | % Variation |
|---|---|---|---|
| mean 123.03 *std. dev. 5.24* | mean 94.80 *std. dev. 19.21* | -28.23 | - 22.9% |

### Example 2 (comparison)

To compare the tightening effect at 5 minutes of a known composition with lifting effect, similarly to example 1, RILASTIL INSTANT SERUM was used in that most recognised by pharmacists as a product with immediate lifting effect and reference product on the market in the industry.

Said product has the following composition (INCI nomenclature): Aqua, Steareth 2, Glycerin, Ppg-15 Stearyl Ether, Sodium Polystyrene Sulfonate, Helianthus Annuus, Steareth-21, Dimethicone, Xantam Gum, Aloe Barbadensis, Serica, Hydrogenated Coco-Glycerides, Lecithin, Glycolipids, Tocopherol, Tocopheryl Acetate, Phospholipids, Octyldodecanol, Imidazolidinyl Urea, Methylparaben, Propylparaben, Disodium Edta, Bha, Parfum, in which the active ingredients are: *Helianthus Annuus, Aloe Barbadensis, Serica, Lecithin, Glycolipids, Tocopherol, Tocopheryl Acetate, Phospholipids.*

Initially the known composition was applied to 3 women, one per age range as defined in example 1, valuating the lifting effect 5 minutes after the application, similarly to what is reported in example 1.

The test was not then performed subsequently on a larger number of people given the smaller decrease of one or of both the parameters of mean and maximum roughness at the end of the treatment on the three women.

### Area around the eyes (women)

### Ra (meal roughness)

| T₀ | T₅ₘᵢₙ | Variation | % Variation |
|---|---|---|---|
| mean 22.06 *std. dev. 1.82* | mean 19.64 *std. dev. 1.67* | -2.42 | -11.0% |

### Rz (maximum roughness)

| T₀ | T₅ₘᵢₙ | Variation | % Variation |
|---|---|---|---|
| mean 95.30 *std. dev. 14.25* | mean 92.47 *std. dev. 12.16* | -2.83 | - 3.0% |

### Area around the lips (women)

### Ra (mean roughness)

| T₀ | T₅ₘᵢₙ | Variation | % Variation |
|---|---|---|---|
| mean 25.62 *std. dev. 1.28* | mean 25.10 *std. dev. 2.53* | - 0.52 | - 2.0% |

### Rz (maximum roughness)

| T₀ | T₅ₘᵢₙ | Variation | % Variation |
|---|---|---|---|
| mean 107.49 *std. dev. 10.77* | mean 106.69 *std. dev. 13.66* | -0.80 | - 0.7% |

### Back of the hands (women)

### Ra (mean roughness)

| T₀ | T₅ₘᵢₙ | Variation | % Variation |
|---|---|---|---|
| mean 26.30 *std. dev. 1.38* | mean 24.62 *std. dev. 1.86* | - 1.68 | -6.4% |

### Rz (maximum roughness)

| T₀ | T₅ₘᵢₙ | Variation | % Variation |
|---|---|---|---|
| mean 114.60 *std. dev. 3.91* | mean 115.78 *std. dev. 8.37* | 1.18 | 1.0% |

The instrumental measurements highlighted the fact that the application of the product in accordance with the invention led, after the same elapsed time (5 minutes), to a significant decrease in the parameters of skin roughness Ra (mean roughness) and Rz (maximum roughness) at the wrinkles around the eyes, the wrinkles around the lips and the back of the hands.

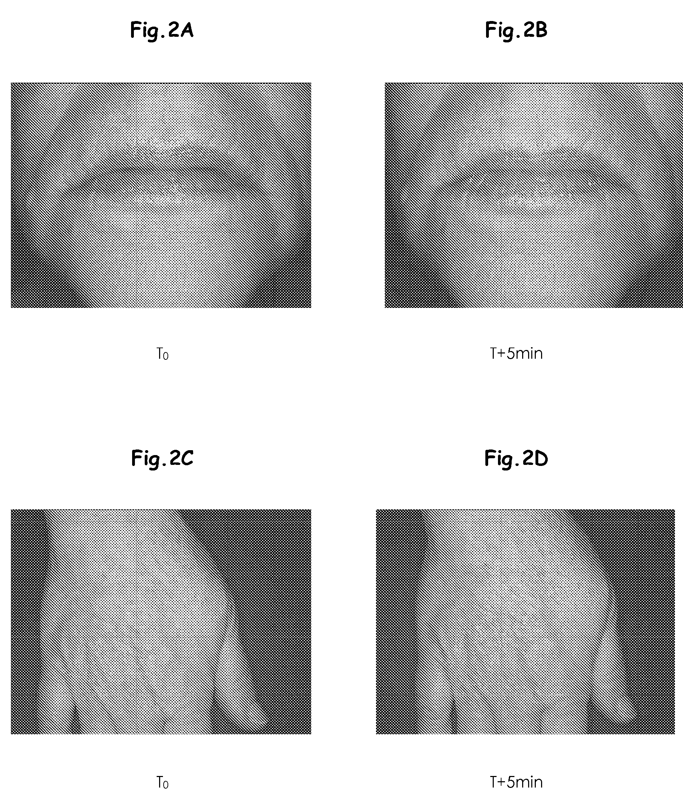

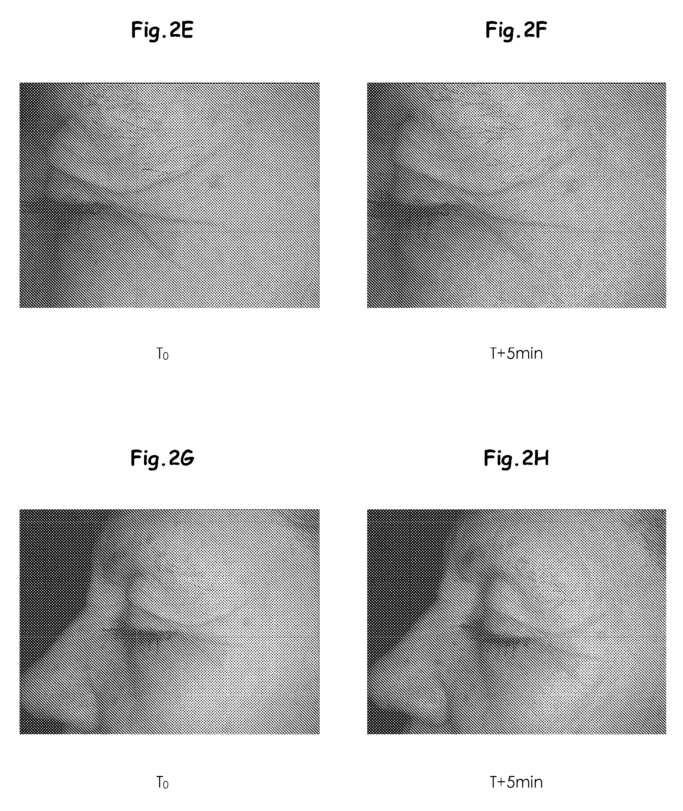

The immediate lifting effect of the product was also highlighted by a visible improvement in the skin roughness which can be clearly appreciated from the digital images of Figs. 1 and 2, where Figure 1 shows skin portions of one subject (number 7) treated with the serum of the invention, while Figure 2 shows skin portions of a subject (number 22) treated with the reference commercial serum.

### Example 3

Example 1 and comparative example 2 were repeated, applying on the face (divided into two halves) of 21 women the product in accordance with the invention (DMC SERUM 7161 A) and the commercial product RILASTIL INSTANT SERUM (7161 B) yet valuating roughness only 3 minutes after the application.

The sample of the women (Caucasian race) was divided up as follows: 7 women aged between 50 and 55 years; 7 women aged between 56 and 60 years; 7 women aged between 61 and 65 years, all having wrinkles in the area around the eyes.

After the basal measurements described in example 1, the operator applied an exact quantity of product according to the following methods:
- apply 2 drops maximum of each product on half face (from the hairline as far as the neck);
- spread the serum over the eyelids, under the eyes, around the lips and in the areas where wrinkles are present;
- apply delicately without rubbing;
- leave the skin to dry for 3 minutes.

As mentioned previously, the anti-wrinkle efficacy is demonstrated by a decrease in one or both of the parameters Ra, Rz at the end of the treatment. The values of the parameters obtained in the test of the present example are given herein below:

### Ra (Mean roughness)

| | T₀ | T₃ₘᵢₙ | Variation | % Variation | t-test T₀ vs T₃ₘᵢₙ |
|---|---|---|---|---|---|
| DMC SERUM 7161 A | mean 27.26 *std. dev. 7.46* | mean 20.88 *std. dev. 6.76* | -6.38 | -23.4% | p < 0.0001 |
| 7161 B | mean 26.13 *std. dev. 9.52* | mean 25.78 *std. dev. 7.78* | -0.35 | -1.3% | p < 0.05 |
| | | | | | |
| **(T₃ₘᵢₙ - T₀) DMC SERUM 7161 A vs (T₃ₘᵢₙ - T₀) 7161 B : p < 0.001** | | | | | |

**DMC SERUM 7161 A:** a significant reduction in the value of mean roughness 3 minutes after application of the product was noted.

7161 B: no variation was noted after application of the product. A significant difference between the two products is therefore evident.

### Rz (maximum roughness)

| | T₀ | T₃ₘᵢₙ | Variation | % Variation | t-test T₀ vs T₃ₘᵢₙ |
|---|---|---|---|---|---|
| **DMC SERUM** **7161 A** | mean 125.50 *std. dev. 27.30* | mean 97.06 *std. dev. 29.61* | -28.44 | **- 22.7%** | **p < 0.0001** |
| **7161 B** | mean 123.35 *std. dev. 35.61* | mean 125.04 *std. dev. 28.15* | 1.69 | 1.4% | p < 0.05 |
| | | | | | |
| **(T₃ₘᵢₙ - T₀) DMC SERUM 7161 A vs (T₃ₘᵢₙ - T₀) 7161 B : p < 0.0001** | | | | | |

**DMC SERUM 7161 A:** a significant reduction in the value of maximum roughness 3 minutes after application of the product was noted.

**7161 B:** no variation was noted after application of the product. A significant difference between the two products is therefore evident.

The instrumental measurements highlighted the fact that the application of the product in accordance with the invention led, after the same elapsed time (3 minutes), to a significant decrease in the parameters of skin roughness Ra (mean roughness) and Rz (maximum roughness) while no variation after the application of RILASTIL INSTANT SERUM was noted. Moreover the digital images showed a visible improvement in the signs of photo-ageing.

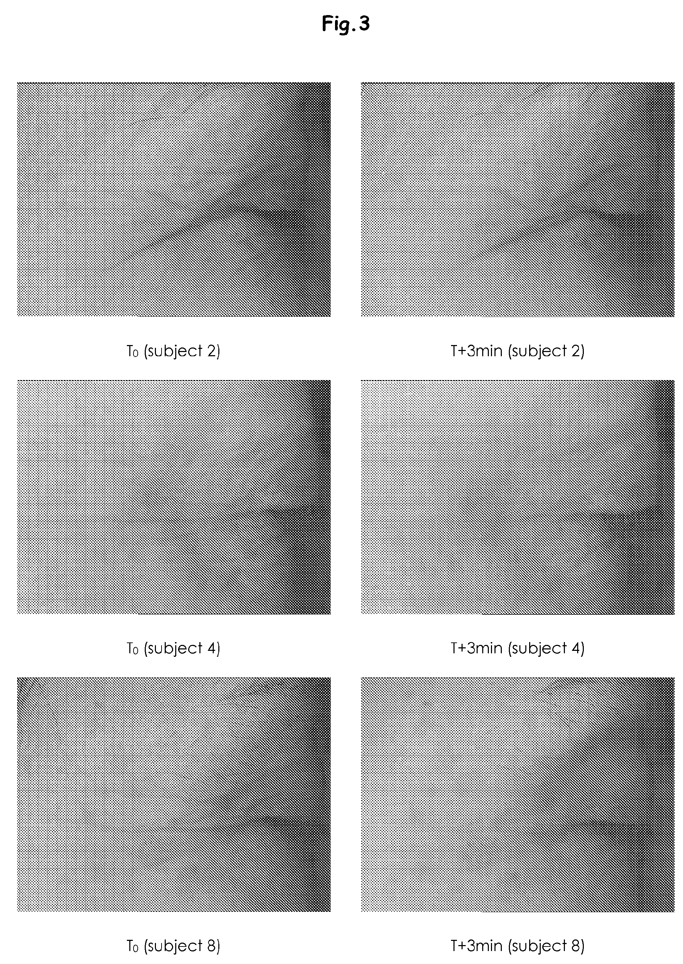

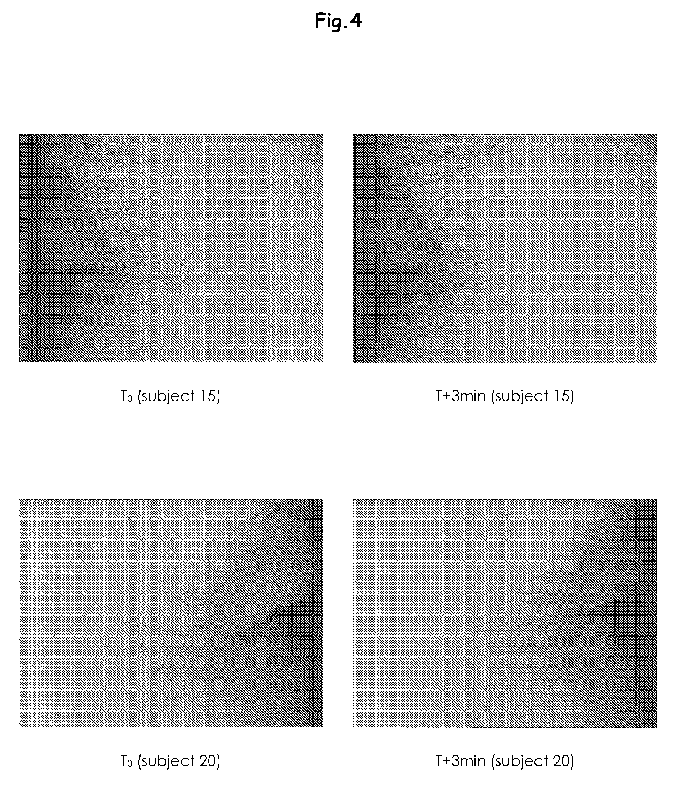

The immediate lifting effect is highlighted by a visible improvement in the skin roughness which can be clearly appreciated from the digital images, attached here as Figs. 3 and 4, which show skin portions of four subjects (numbers 2, 4, 8, 15, 20) before and after treatment with the serum of the invention.

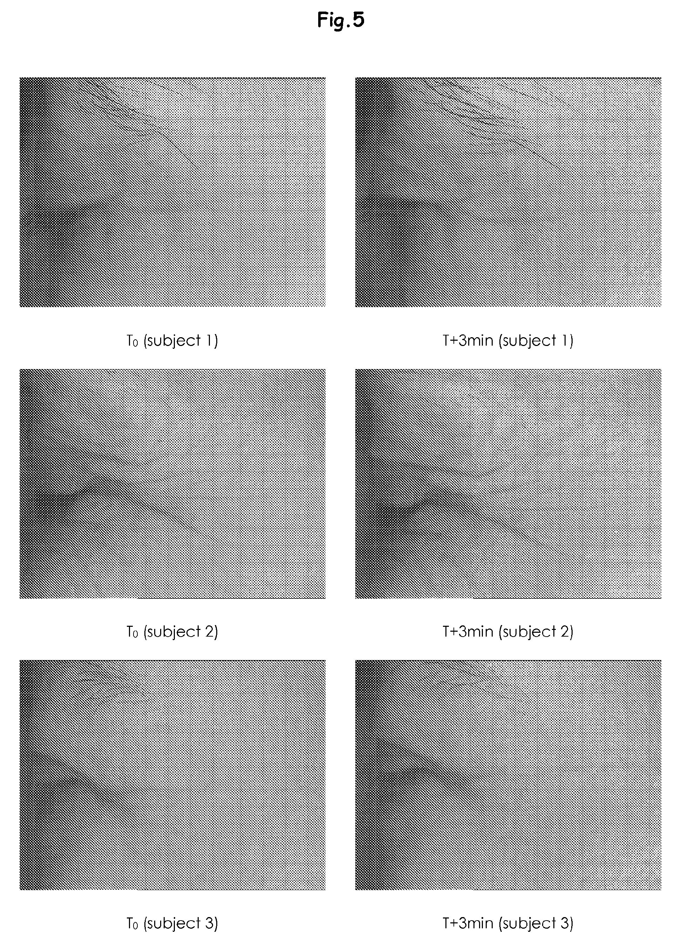

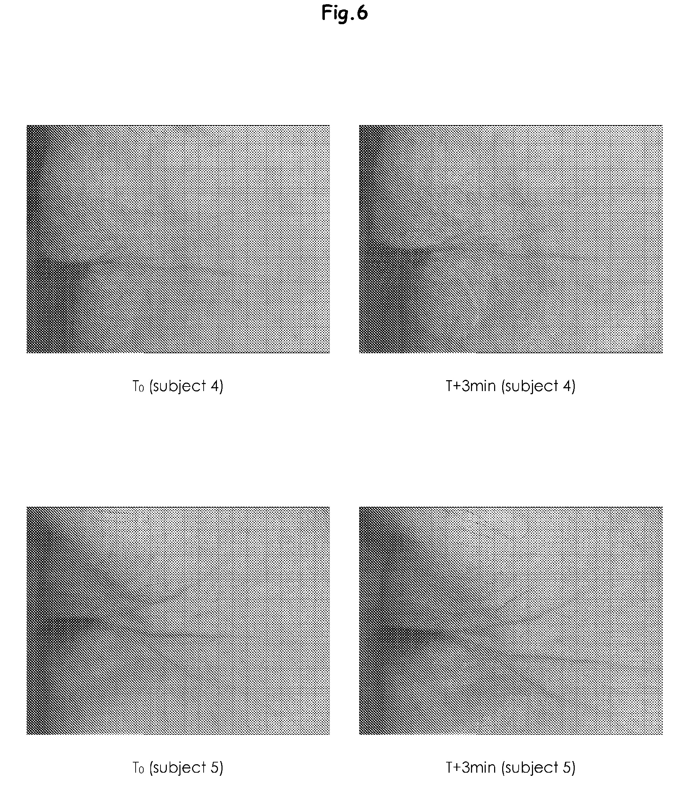

Figures 5 and 6 show skin portions of four subjects (numbers 1, 2, 3, 4, 5) before and after treatment with the commercial serum RILASTIL.

## Claims

1. Process for preparing a dermo-cosmetic semifluid aqueous serum/gel with instant lifting effect having a composition comprising, as main active ingredients, vitamin A, vitamin E, pure or in its other forms, collagen, ascorbic acid in combination with a mixture of plant extracts of *Spilanthes Acmella Flower, Cucumus Sativus, Olea Europaea Leaf, Punica Granatum, Gingko Biloba, vitis Vinifera Leaf,* in at least one physiologically acceptable medium or carrier, preferably water,
said process comprising the following operations:
- preparation of a colloidal gel from a powdery mixture of *Cellulose Gum* and *Magnesium Aluminum Silicate*
- addition to this colloidal gel, while stirring, of
a) a mixture comprising vitamin A, vitamin E, pure or in its other forms, extract of *Spilanthes Acmella Flower,* dissolved in a solvent formed by a mixture of propylene glycol, ethylparaben and methylparaben,
b) an aqueous solution of ascorbic acid,
c) an aqueous dispersion of *Sodium Silicate,*
- addition of the remaining active ingredients.

2. Process according to claim 1, wherein said operations are constituted by the following steps:
STEP 1:
preparation of a homogeneous powdery mixture formed by *Cellulose Gum* and *Magnesium Aluminum Silicate,* operating in such a manner as to avoid humidity absorption, even partially;
STEP 2:
addition, slowly and while stirring, of said powdery mixture of STEP 1 to a first portion of water brought to a temperature of 70°-80°, until a colloidal gel is obtained;
STEP 3:
preparation, while stirring, of a mixture of *Propylene Glycol* with ethylparaben and methylparaben, at a temperature between 40-60°, until obtaining a complete solubilisation of the abovementioned components, and cooling of the mixture to ambient temperature;
STEP 4:
addition to the mixture of STEP 3 of each of the following components while constantly stirring:
- vitamin E, pure or in its other forms, preferably *Tocopheryl Acetate,*
- vitamin A, preferably *Retinyl Palmitate,*
- mixture of *Caprylic*/*Capric Triglyceride,*
- *Spilanthes Acmella Flower* extract,
STEP 5:
cooling of the colloidal gel of STEP 2 to ambient temperature and adding to it, while constantly stirring, of STEP 4;
STEP 6:
preparation of an aqueous solution of *Ascorbic Acid,* and adding of said solution to STEP 2, while constantly stirring;
STEP 7:
adding of *Sodium Silicate* to a second portion of water having a temperature of 35-40°;
STEP 8:
Adding, while constantly stirring, of the mixture of STEP 7 to STEP 2;
STEP 9:
Adding slowly to the so obtained STEP 2, while stirring and under vacuum, preferably high vacuum, of the following components:
- *Soluble Collagen,*
- *Cucumis Sativus Extract,*
- *Vitis Vinifera Leaf Extract,*
- *Ginkgo Biloba Extract,*
- *Olea Europaea Leaf Extract,*
- *Punica Granatum Extract.*

3. Process according to claim 2, wherein the first portion of water used in STEP 2 represents an amount between 70% and 80% with respect to the total amount of water in the final composition.

4. Process according to claim 2 or 3, wherein the second portion of water used in STEP 7 is an amount comprised between 10% and 20% with respect to the total amount of water in the final composition.

5. Process according to any one of previous claims 2 to 4, wherein in STEP 4 each component is added after the solubilisation of the previously added components.

6. Process according to any one of the previous claims, wherein the following components (*INCI nomenclature*) are added in the following amounts (% by weight with respect to the total weight of the composition):
- vitamin E, pure or in its other forms, preferably *Tocopheryl Acetate,* in amounts between 0.05% and 0.15%, preferably between 0.1 and 0.15%; and/or
- vitamin A (*Retinyl Palmitate*) in amounts between 0.08 and 0.20%, preferably between 0.08 and 0.14%; and/or
- *Ascorbic Acid* in amounts between 0.08 and 0.15%, preferably between 0.09 and 0.14%; and/or
- *Soluble Collagen* in amounts between 0.08 and 0,15%, preferably between 0.09 and 0.15%.

7. Process according to any one of the previous claims, wherein the following components (*INCI nomenclature*) are added in the following amounts (% by weight with respect to the total weight of the composition):
- *Spilanthes Acmella Flower Extract,* in amounts between 0.001 and 0.020%, preferably between 0.001% and 0.002%; and/or
- *Cucumis Sativus Extract,* in amounts between 0.01 and 0.15%, preferably between 0.09 and 0.15%; and/or
- *Vitis Vinifera Leaf Extract,* in amounts between 0.01 and 0.10%, preferably between 0.03 and 0.10%; and/or
- *Ginkgo Biloba Extract,* in amounts between 0.01 and 0.15%, preferably between 0.05% and 0.15%; and/or
- *Olea Europaea Leaf Extract,* in amounts between 0.01 and 0,2%; and/or
- *Punica Granatum Extract,* in amounts between 0.01 and 0.2%, preferably between 0.07 and 0.18%.

8. Process according to any one of the previous claims, wherein the following components (*INCI nomenclature*) are added in the following amounts (% by weight with respect to the total weight of the composition):
- *Caprylic*/*Capric Triglyceride,* in amounts between 0.001 and 0.020%, preferably between 0.001% and 0.002%;
- *Cellulose Gum,* in amounts between 0.7% and 1.5%;
- *Magnesium Aluminum Silicate,* in amounts between 0.5-1.50%, preferably between 0.8% and 1.3;
- *Propylene Glycol,* in amounts between 6% and 8%, preferably between 7.5% and 8%;
- *Ethylparaben,* in amounts between 0.05-0.15%, preferably between 0.08 and 0.13%;
- *Methylparaben* in amounts between 0.15 and 0.3%, preferably between 0.15 and 0.25%;
- *Sodium Silicate,* in amounts between 3 and 5.50%, preferably 4 and 5.50%.

9. Dermo-cosmetic composition having an instant lifting effect comprising, as main active ingredients, vitamin A, vitamin E, pure or in its other forms, in particular tocopheril acetate, collagen, ascorbic acid in combination with a mixture of plant extracts of *Spilanthes Acmella Flower; Cucumus Sativus, Olea Europaea Leaf, Punica Granatum, Gingko Biloba, Vitis Vinifera Leaf,* in at least one physiologically acceptable medium or carrier, preferably water, obtained from the process as defined in any one of the previous claims 1-8.

10. Dermo-cosmetic composition according to claim 9, wherein
- vitamin E, pure or in its other forms, preferably *Tocopheryl Acetate,* in amounts between 0.05% and 0.15%; and/or
- vitamin A (*Retinyl Palmitate*) in amounts between 0.08 and 0.20%; and/or
- *Ascorbic Acid* in amounts between 0.08 and 0.15%; and/or
- *Soluble Collagen* in amounts between 0.08 and 0.15%; and/or
- *Spilanthes Acmella Flower Extract,* in amounts between 0.001 and 0.020%; and/or
- *Cucumis Sativus Extract,* in amounts between 0.01 and 0.15%; and/or
- *Vitis Vinifera Leaf Extract,* in amounts between 0.01 and 0.10%; and/or
- *Ginkgo Biloba Extract,* in amounts between 0.01 and 0.15%; and/or
- *Olea Europaea Leaf Extract,* in amounts between 0.01 and 0.2%; and/or
- *Punica Granatum Extract,* in amounts between 0.01 and 0.2%.

11. Dermo-cosmetic composition according to claim 10, comprising moreover
- *Caprylic-Capric Triglyceride,* in amounts between 0.001 and 0.020%; and/or
- *Cellulose Gum,* in amounts between 0.7% and 1.5%; and/or
- *Magnesium Aluminum Silicate,* in amounts between 0.5-1.50%; and/or
- *Propylene Glycol,* in amounts between 6% and 8%; and/or
- *Ethylparaben,* in amounts between 0.05-0.15%; and/or
- *Methylparaben* in amounts between 0.15 and 0.3%; and/or
- *Sodium Silicate,* in amounts between 3 and 5.50%.

12. Composition according to any one of claims 9 to 11, having the following formulation (% by weight with respect to the total weight of the composition):
| | |
|---|---|
| *Aqua* | 84.20280 |
| *Propylene Glycol* | 7.80000 |
| *Sodium Silicate* | 5.28000 |
| *Cellulose Gum* | 0.85000 |
| *Magnesium Aluminum Silicate* | 0.80000 |
| *Methylparaben* | 0.20000 |
| *Ascorbic Acid* | 0.09500 |
| *Cucumis Sativus Extract* | 0.01400 |
| *Ethylparaben* | 0.09000 |
| *Retinyl Palmitate* | 0.08000 |
| *Tocopheryl Acetate* | 0.15000 |
| *Glycerin* | 0.08000 |
| *Caprylic*/*Capric Triglyceride* | 0.00120 |
| *Ginkgo Biloba Extract* | 0.05500 |
| *Olea Europaea Leaf Extract* | 0.01100 |
| *Punica Granatum Extract* | 0.11000 |
| *Vitis Vinifera Leaf Extract* | 0.09000 |
| *Soluble Collagen* | 0.09000 |
| *Spilanthes Acmella Flower Extract* | 0.00100 |
